**Europäisches Patentamt**

**European Patent Office**

**Office européen des brevets**

(19)

(11) **EP 1 241 132 A2**

(12) # EUROPÄISCHE PATENTANMELDUNG

(43) Veröffentlichungstag:
**18.09.2002 Patentblatt 2002/38**

(51) Int Cl.⁷: **C01B 33/18**, C01B 33/193,
A61K 7/16

(21) Anmeldenummer: **02000632.6**

(22) Anmeldetag: **11.01.2002**

(84) Benannte Vertragsstaaten:
**AT BE CH CY DE DK ES FI FR GB GR IE IT LI LU
MC NL PT SE TR**
Benannte Erstreckungsstaaten:
**AL LT LV MK RO SI**

(30) Priorität: **16.03.2001 DE 10112650**

(71) Anmelder: **Degussa AG
40474 Düsseldorf (DE)**

(72) Erfinder:
• **Uhrlandt, Stefan, Dr.
53859 Niederkassel (DE)**
• **Schmoll, Ralf, Dr.
53125 Bonn (DE)**
• **Storeck, Arnold, Dr.
60322 Frankfurt am Main (DE)**

(54) **Inhomogene Kieselsäuren in Zahnpflegemittel**

(57)    Die Erfindung betrifft Kieselsäuren mit inhomogener Struktur bzw. Aufbau, Verfahren zu deren Herstellung
und deren Verwendung in Zahnpflegemitteln.

**EP 1 241 132 A2**

**Beschreibung**

Die Erfindung betrifft Kieselsäuren mit inhomogener Struktur bzw. Aufbau, Verfahren zu deren Herstellung und deren Verwendung in Zahnpflegemittel.

Leicht dispergierbare Kieselsäuren werden z. B. gemäß EP 0 901 986 oder EP 0 647 591 durch Fällung von Wasserglas mit Schwefelsäure und anschließender Trocknung hergestellt. Die getrockneten Produkte werden anschliessend vermahlen und/oder granuliert.

In einem anderen Verfahren werden Kieselsäuren ebenfalls durch saure Fällung hergestellt, jedoch mittels Verdüsung in Heißluft getrocknet und gleichzeitig zu leicht zerstörbaren Kugeln geformt. So beschreibt EP 018 866 die Herstellung von sprühgetrockneter Kieselsäure mit einem mittleren Partikeldurchmesser von über 80 µm, wobei die Partikel eine homogene Struktur besitzen und massiv sind.

Für die Anwendung in Zahnpflegemitteln sind neben Verdickungswirkung und Abrasivität die spezifischen Oberflächen (BET, CTAB) und die Ölaufnahmekapazität (DBP) von Kieselsäuren wichtig.

Da nur spezielle, d. h. aufwendig herzustellende Kieselsäuren gleichzeitig ausreichende Verdickungswirkung und Abrasivitäten aufweisen, werden in Zahnpflegemitteln meist zwei verschiedene Kiesensäuretypen eingesetzt.

Es wäre wünschenswert, eine Kieselsäure herzustellen, die gleichzeitig breite Bereiche von physikalisch-chemischen Daten wie BET-, oder CTAB-Oberfläche, und gute Abrasivität verbunden mit einer guten Verdickungswirkung abdeckt.

Überraschenderweise wurde gefunden, dass eine Kieselsäure, die eine inhomogene Zusammensetzung aufweist, leicht auf die geforderten Anforderungen eingestellt werden kann.

Zusätzlich sind erfindungsgemäß hergestellte Kieselsäuren staubarm, was die Handhabung erleichtert und das "Verstauben" der Anlagen beim Kunden verhindert.

Gegenstand der vorliegenden Erfindung sind daher Kieselsäuren, enthaltend mindestens zwei Kieselsäurefraktionen, die sich in mindestens einem Meßwert für BET-Oberfläche, CTAB-Oberfläche und DBP-Aufnahme um mindestens 10 % unterscheiden.

Die erfindungsgemäßen Kieselsäuren sind daher besonders als Füllstoff in Zahnpflegemitteln geeignet.

Der Aufbau der Kieselsäuren aus mindestens zwei Kieselsäurefraktionen bewirkt eine Inhomogenität der Kieselsäure, die sich in gleichzeitig guten Abrasivitäten und Verdickungswirkung sowie einem geringen Feinanteil niederschlägt.

Die Abrasivwerte gemäß RDA-Methode bewegen sich im für moderne Zahnpastenkieselsäuren typischen Bereich von 45% bis 70%, können diesen aber auch über oder unterschreiten, abhängig von der gewählten Mischung der Fällsuspensionen und deren verfahrenstechnischer Aufbereitung. Ebenfalls im typischen Fließbereich sind die daraus hergestellten Zahnpasten. Sie haben nämlich im Schubspannungsversuch bis zu einer Schubspannung von 600 Pa eine Schwergeschwindigkeit von 100 s$^{-1}$ bis 500 s$^{-1}$, abhängig von der eingesetzten erfindungsgemäßen Kieselsäuremischung und der in der Paste verwendeten Konzentration. Höhere oder niedrigere Schwergeschwindigkeiten im genannten Schubspannungsbereich können abhängig von der gewählten Mischung der Fällsuspensionen und deren verfahrenstechnischer Aufbereitung auftreten.

Erfindungsgemäße Kieselsäuren besitzen einen Feinanteil von höchstens 10 % mit Teilchendurchmesser kleiner oder gleich 63 µm (Alpine Siebrückstand).

Ein ähnliches Konzept, d. h. inhomogene Kieselsäuren, wird in EP 0 942 029 verfolgt. Hier werden Gummi-Kompositionen beschrieben, die eine Fällungskieselsäure in zwei verschiedenen Aggregatgrößen enthält. Die verschiedenen Aggregatgrößen werden für die leichte Dispergierbarkeit der Kieselsäure in der Gummimischung eingesetzt.

Die unterschiedlichen Kieselsäurefraktionen der vorliegenden Erfindung sind nicht beschrieben; außerdem ist eine unterschiedliche Aggregatgröße der Kieselsäurefraktionen im vorliegenden Fall von nebengeordneter Bedeutung, wichtig sind die Unterschiede der physikalisch-chemischen Daten.

"Kieselsäurefraktion" im Sinne der vorliegenden Erfindung bezeichnet verschiedene Sorten von Kieselsäuren, die aufgrund verschiedener Herstellungsverfahren oder -Varianten einen Unterschied von 10 % in mindestens einer der o. g. physikalisch-chemischen Daten aufweisen. Bevorzugt weisen zwei, besonders bevorzugt drei dieser Parameter einen solchen Unterschied auf.

Die Unterschiede in den bereits genannten Parametern können durch unterschiedliche Herstellverfahren der Kieselsäurefraktionen erhalten werden. So können alle, ein oder mehrere der Kieselsäurefraktionen Fällungskieselsäuren und/oder pyrogene Kieselsäuren sein. Bei Fällungskieselsäuren ist es vor allem möglich, durch verschiedene Fällungsverfahren verschiedene Kieselsäurefraktionen zu erhalten. Erfindungsgemäße Kieselsäuren können auch aus Fraktionen von gefällten und pyrogenen Kieselsäuren hergestellt werden.

Für Fällungskieselsäuren sind verschiedene Fällmethoden bekannt und können z. B. in EP 0 901 986, EP 0 937 755, EP 0 643 015 oder EP 0 647 591 nachgelesen werden. In den Beispielen sind exemplarisch zwei Fällungskieselsäuren aus verschiedenen Herstellverfahren zur erfindungsgemäßen inhomogenen Kieselsäure verarbeitet worden.

Die Kieselsäurefraktionen können Fällungskieselsäuren oder pyrogene Kieselsäuren sein, wobei eine Mischung der Fraktionen an verschiedenen Prozeßschritten, die üblicherweise bei der Herstellung von Kieselsäuren durchgeführt werden, erfolgen kann.

Bei Verwendung von Fraktionen aus Fällungskieselsäuren kann die Mischung nach der Fällung von Silikat mit einer Säure (in der Regel Wasserglas, d. h. Natriumsilikat mit Schwefelsäure) durch Vermischen der Fällungssuspensionen oder der nach Filtration der Suspensionen erhaltenen Filterkuchen sowie verflüssigten, resuspendierten Filterkuchen erfolgen. Es ist auch möglich, bereits hergestellte und getrocknete Kieselsäurefraktionen als Feststoff den Suspensionen oder den Filterkuchen zuzusetzen.

Die so erhaltenen Mischungen müssen ggf. abfiltriert und in üblicher Weise getrocknet werden. Trocknungsverfahren sind z. B. Sprühtrocknung, Düsenturm, Etagentrockner, Drehrohrtrockner oder Spin-Flash-Trockner.

Nach dem Trocknen ist eine abschließende Vermahlung und/oder Granulation möglich.

Es ist auch möglich, die Kieselsäurefraktionen im trockenen Zustand zu vermischen. Hierzu kann sich eine Resuspendierung mit den o. g. Trocknungsschritten anschließen und/oder eine Vermahlung/Granulation.

Erfindungsgemäße Kieselsäuren können die folgenden physikalisch-chemischen Daten aufweisen:

BET-Oberfläche          30 - 300 $m^2$/g, insbesondere 30 - 200 $m^2$/g
CTAB-Oberfläche         30 - 300 $m^2$/g, insbesondere 30 - 200 $m^2$/g
DBP-Aufnahme            80 - 300 g/100 g.

Diese physikalisch-chemischen Daten betreffen die Kieselsäure gemäß der Erfindung <u>als solche,</u> und nicht die Kieselsäurefraktionen.

Die physikalisch-chemischen Daten der Kieselsäurefraktionen müssen sich in beschriebener Weise um mindestens 10, bevorzugt um mindestens 15, besonders bevorzugt um mindestens 20 % unterscheiden.

Die physikalisch-chemischen Daten werden mit folgenden Methoden bestimmt:

BET-Oberfläche          Areameter, Fa. Ströhlein, gemäß ISO 5794/Annex D
CTAB-Oberfläche         bei pH 9, gemäß Jay, Janzen und Kraus in "Rubber Chemistry and Technology" 44 (1971) 1287
DBP-Zahl                ASTM D 2414-88

Ein weiterer Gegenstand der Erfindung ist ein Verfahren zur Herstellung von Kieselsäuren, enthaltend mindestens zwei Kieselsäurefraktionen, wobei mindestens zwei Kieselsäurefraktionen, die sich in mindestens einem Meßwert für BET-Oberfläche, CTAB-Oberfläche und DBF-Aufnahme um mindestens 10 % unterscheiden, miteinander vermischt werden.

Der Anteil der jeweiligen Fraktionen in der Suspension bzw. an der Kieselsäure sollte jeweils zwischen 5 und 95 Gew.-%, bezogen auf die trockene Kieselsäure, betragen.

Die Kieselsäure wird bevorzugt in Partikelform mit einem mittleren Durchmesser von über 80, insbesondere über 100, besonders bevorzugt über 200 μm, z. B. durch die Sprühtrocknung erhalten. Die Sprühtrocknung der Suspension kann z. B. gemäß US 4 097 771 durchgeführt werden.

Die erfindungsgemäßen Kieselsäuren können daher als Zahnpflegemitteln, Zahnpasten oder Schleifmitteln verwendet werden.

Weiterhin können die erfindungsgemäßen Kieselsäuren in allen Anwendungsgebieten verwendet werden, in denen üblicherweise Kieselsäuren eingesetzt werden, wie z. B. in Batterieseparatoren, Anti-Blocking-Mittel, Mattierungsmittel in Lacken, Papierstrichen oder Entschäumer.

Die Bestimmung des Alpine-Siebrückstandes wird wie folgt durchgeführt, wobei jeweils ein Sieb mit der angegebenen Maschenweite (63 μm, 250 μm) verwendet wird.

<u>Durchführung der Bestimmung des Alpine-Siebrückstandes:</u>

Zur Bestimmung des Siebrückstandes wird die Kieselsäure- oder Silikat-Probe durch ein 500 μm Sieb abgesiebt, um eventuell vorhandene Entlüftungsagglomerate zu zerstören. Dann werden 10 g der gesiebten Probe auf das Luftstrahlsieb gegeben, das mit einem 63 μm Siebgewebe bestückt ist und bei 200 mm Wassersäule-Unterdruck abgesiebt. Kieselsäureoder Silikatpartikel, die sich am Siebdeckel des Gerätes absetzen, werden durch vorsichtiges Schlagen auf den Knopf des Siebdeckels abgeklopft. Der Siebvorgang dauert im allgemeinen 5 Minuten. Er ist beendet, wenn der Rückstand konstant bleibt, was meistens am rieselfähigen Aussehen zu erkennen ist. Zur Sicherheit siebt man dann noch eine weitere Minute.

Bei Agglomeraten, die sich eventuell bilden, wird der Siebvorgang kurz unterbrochen und die Agglomerate mit einem Pinsel unter leichtem Druck zerstört. Nach der Siebung wird der Siebrückstand vorsichtig vom Luftstrahlsieb geklopft und zurückgewogen. Der Siebrückstand wird in Prozenten angegeben, immer in Verbindung mit der Maschenweite des Siebes.

Berechnung:

$$\% \text{ Siebrückstand} = \frac{A \cdot 100}{E}$$

A = Auswaage in g
E = Einwaage in g

Geräte

Alpine Luftstrahlsieb, Labortyp S 200
Staubsauger oder Gebläse
Luftstrahlsieb mit Siebgewebe 63 μm nach DIN 4188
Präzisionswaage

Die folgenden Beispiele sollen die Erfindung näher erläutern, ohne ihren Umfang zu beschränken.
Es wurden zwei Kieselsäurefraktionen A gemäß US 1 043 282, DE 24 47 613 und B gemäß DE 44 23 493 hergestellt und die aus den Fällungen erhaltenen Suspensionen in der im folgenden beschriebenen Weise weiter umgesetzt.

**Beispiel 1**

Die Fällsuspensionen der Kieselsäurefraktionen A und B wurden im Verhältnis 50:50 gemischt. Dazu wurde 80 kg der Fällungskieselsäure A (Feststoffgehalt ca. 170 g/l) mit 80 kg der Fällungskieselsäure B (Feststoffgehalt ca. 63 g/l) in einem gerührten Behälter gemischt. Die erhaltenen Mischung wurde filtriert, der Filterkuchen mit etwas Säure verflüssigt und auf einem Düsenturmtrockner versprüht. Die Analysendaten sind in Tabelle 1 zusammengefaßt.

**Beispiel 2**

Die Fällsuspensionen der Fällungskieselsäuren A und B wurden im Verhältnis 70:30 gemischt. Dazu wurde 196 kg der Fällungskieselsäure A (Feststoffgehalt ca. 174 g/l) mit 84 kg der Fällungskieselsäure B (Feststoffgehalt ca. 63 g/l) in einem gerührten Behälter gemischt. Die erhaltene Mischung wurde filtriert, der Filterkuchen mit etwas Säure verflüssigt und auf einem Düsenturmtrockner versprüht. Die Analysendaten sind in Tabelle 1 zusammengefaßt.

**Beispiel 3**

Die Fällsuspensionen der Fällungskieselsäuren A und B wurden im Verhältnis 30:70 gemischt. Dazu wurde 71 kg der Fällungskieselsäure A (Feststoffgehalt ca. 174 g/l) mit 142 kg der Fällungskieselsäure B (Feststoffgehalt ca. 63 g/l) in einem gerührten Behälter gemischt. Die erhaltene Mischung wurde filtriert, der Filterkuchen mit etwas Säure verflüssigt und auf einem Düsenturmtrockner versprüht. Die Analysendaten sind in Tabelle 1 zusammengefaßt.

**Beispiel 4**

Es wurde eine Mischung der getrockneten Kieselsäurefraktionen (50:50) hergestellt.

**Beispiel 5**

Gemäß nachstehender Rezeptur wurde eine weiße, opake Zahnpasta mit einer Mischung der Fällungskieselsäuren gemäß Beispiel 3 (Verhältnis A zu B wie 30:70) hergestellt.

| Substanz | Masse [%] |
|---|---|
| Kieselsäure gemäß Beispiel 3 | 20,00 |
| Sorbitol, 70%ige Lösung | 40,00 |

(fortgesetzt)

| Substanz | Masse [%] |
|---|---|
| Wasser | 31,69 |
| Polyethylenglykol 400 | 3,50 |
| Natriumlaurylsulfat | 1,20 |
| Carboxymethylcellulose | 1,20 |
| Natriummonofluorsphosphat | 0,76 |
| Titandioxid | 0,40 |
| Methylparaben, Natriumsalz | 0,15 |
| Sacharin, Natriumsalz | 0,10 |
| Aroma | 1,00 |

Um eine gute Homogenisierung sicherzustellen, wurde die Paste nach ihrem Ansatz im Mischer mehrfach mittels eines Dreiwalzenstuhles mechanisch bearbeitet.

**Beispiel 6**

Gemäß nachstehender Rezeptur wurde eine transparente Zahnpasta mit einer Mischung der Fällungskieselsäuren gemäß Beispiel 3 (Verhältnis A zu B wie 30:70) hergestellt.

| Substanz | Masse [%] |
|---|---|
| Kieselsäure gemäß Beispiel 3 | 20,00 |
| Glyzerin, 99%ig | 20,00 |
| Sorbitol, 70%ige Lösung | 40,00 |
| Wasser | 12,09 |
| Polyethylenglykol 400 | 3,50 |
| Natriumlaurylsulfat | 1,30 |
| Carboxymethylcellulose | 0,60 |
| Natriummonofluorsphosphat | 0,76 |
| Farbstoff FD&C Nr. 1, 1%ige Lösung | 0,50 |
| Methylparaben, Natriumsalz | 0,15 |
| Sacharin, Natriumsalz | 0,10 |
| Aroma | 1,00 |

Um eine gute Homogenisierung sicherzustellen, wurde die Paste nach ihrem Ansatz im Mischer mehrfach mittels eines Dreiwalzenstuhles mechanisch bearbeitet.

**Tabelle 1:**

Vergleich der Analysendaten aus den Beispielen 1-3 und der Kieselsäurefraktionen A und B:

| | | Kieselsäure-fraktion A | Kieselsäure-fraktion B | Unterschiede der Fraktionen A : B in % | Beispiel 1 | Beispiel 2 | Beispiel 3 | Beispiel 4 |
|---|---|---|---|---|---|---|---|---|
| Glühverlust DIN | % | 5,0 | 3,0 | 40 | 3,7/7,4 | 2,7/7,0 | 3,4/8,0 | 3,9/7,3 |
| Wassergehalt | % | 5,0 | 5,0 | 0 | 3,9 | 4,5 | 4,7 | 3,6 |
| pH-Wert /A. Z. | | 6,5 | 6,9 | 5,7 | 6,2 | 6,1 | 6,3 | 6,7 |
| Leitfähigkeit | $\mu$S | 800 | 380 | 52,5 | 440 | 290 | 300 | 480 |
| BET-Oberfläche | $m^2/g$ | 195 | 45 | 76,9 | 113 | 89 | 146 | 114 |
| CTAB- Oberfläche | $m^2/g$ | 175 | 40 | 77,1 | 106 | 81 | 132 | 110 |
| DBP-Aufnahme | g/100g | 270 | 120 | 55,5 | 185 | 156 | 203 | 193 |
| Stampfdichte | g/l | 90 | 430 | 79,1 | 334 | 392 | 385 | 175 |
| Alpine Siebrückstand 63 $\mu$m | % | n.b. | n.b. | | 93 | 63 | 98 | 0,2 |
| Alpine Siebrückstand 180 $\mu$m | % | n.b. | n.b. | | 29 | 0,6 | 74 | 0,02 |
| Alpine Siebrückstand 250 $\mu$m | % | n.b. | n.b. | | 0,8 | 0,2 | 27 | |
| Mittl. TG | $\mu$m | | | | | | | 12,1 |

**Patentansprüche**

1. Kieselsäure, enthaltend mindestens zwei Kieselsäurefraktionen,
**dadurch gekennzeichnet,**
**dass** die mindestens zwei Kieselsäurefraktionen sich in mindestens einem Meßwert für BET-Oberfläche, CTAB-Oberfläche und DBP-Aufnahme um mindestens 10 % unterscheiden.

2. Kieselsäure nach Anspruch 1,
**dadurch gekennzeichnet,**
**dass** sie in Form von Partikeln mit einem mittleren Teilchendurchmesser von über 80 µm vorliegt.

3. Kieselsäure nach einem der Ansprüche 1 oder 2,
**dadurch gekennzeichnet,**
**dass** die Kieselsäure die folgenden physikalisch-chemischen Daten aufweist:

BET-Oberfläche      30 - 300 m$^2$/g
CTAB-Oberfläche     30-300 m$^2$/g
DBP-Aufhahme        80 - 300 g/100 g.

4. Kieselsäure nach einem der Ansprüche 1 bis 3,
**dadurch gekennzeichnet,**
**dass** der jeweilige Anteil einer Kieselsäurefraktion in der Kieselsäure zwischen 5 und 95 Gew.-% liegt.

5. Kieselsäure nach einem der Ansprüche 1 bis 4,
**dadurch gekennzeichnet,**
**dass** eine oder mehrere Kieselsäurefraktionen aus einer Fällungskieselsäure bestehen.

6. Kieselsäure nach einem der Ansprüche 1 bis 5,
**dadurch gekennzeichnet,**
**dass** die Kieselsäurefraktionen durch Fällung von Silikat mit einer Säure hergestellt und die so erhaltenen Fällungssuspensionen gemischt werden.

7. Kieselsäure nach einem der Ansprüche 1 bis 5,
**dadurch gekennzeichnet,**
**dass** die Kieselsäurefraktionen durch Fällung von Silikat mit einer Säure hergestellt, die Fällungssuspension abfiltriert und die so erhaltenen Filterkuchen gemischt werden.

8. Kieselsäure nach einem der Ansprüche 1 bis 5,
**dadurch gekennzeichnet,**
**dass** die Kieselsäurefraktionen durch Fällung von Silikat mit einer Säure hergestellt, die Filterkuchen oder bereits getrocknete Kieselsäure verflüssigt und die so erhaltenen Suspensionen gemischt werden.

9. Kieselsäure nach einem der Ansprüche 1 bis 4,
**dadurch gekennzeichnet,**
**dass** eine oder mehrere Kieselsäurefraktionen aus einer pyrogenen Kieselsäure bestehen.

10. Kieselsäure nach einem der Ansprüche 1 bis 5 und 9,
**dadurch gekennzeichnet,**
**dass** die Kieselsäurefraktionen im trockenen Zustand gemischt werden.

11. Verfahren zur Herstellung von Kieselsäuren, enthaltend mindestens zwei Kieselsäurefraktionen,
**dadurch gekennzeichnet,**
**dass** mindestens zwei Kieselsäurefraktionen, die sich in mindestens einem Meßwert für BET-Oberfläche, CTAB-Oberfläche und DBP-Aufnahme um mindestens 10 % unterscheiden, miteinander vermischt werden.

12. Verfahren nach Anspruch 11,
**dadurch gekennzeichnet,**
**dass** die Kieselsäure in Form von Partikeln mit einem mittleren Teilchendurchmesser von über 80 µm vorliegt.

**13.** Verfahren nach einem der Ansprüche 11 oder 12,
**dadurch gekennzeichnet,**
**dass** die Kieselsäure die folgenden physikalisch-chemischen Daten aufweist:

BET-Oberfläche    30 - 300 m²/g
CTAB-Oberfläche   30 - 300 m²/g
DBP-Aufnahme    80 - 300 g/100 g.

**14.** Verfahren nach einem der Ansprüche 11 bis 13,
**dadurch gekennzeichnet,**
**dass** der jeweilige Anteil einer Kieselsäurefraktion in der Kieselsäure zwischen 5 und 95 Gew.-% liegt.

**15.** Verfahren nach einem der Ansprüche 11 bis 14,
**dadurch gekennzeichnet,**
**dass** eine oder mehrere Kieselsäurefraktionen aus einer Fällungskieselsäure bestehen.

**16.** Verfahren nach einem der Ansprüche 11 bis 15,
**dadurch gekennzeichnet,**
**dass** die Kieselsäurefraktionen durch Fällung von Silikat mit einer Säure hergestellt und die so erhaltenen Fällungssuspensionen gemischt werden.

**17.** Verfahren nach einem der Ansprüche 11 bis 15,
**dadurch gekennzeichnet,**
**dass** die Kieselsäurefraktionen durch Fällung von Silikat mit einer Säure hergestellt, die Fällungssuspension abfiltriert und die so erhaltenen Filterkuchen gemischt werden.

**18.** Verfahren nach einem der Ansprüche 11 bis 15,
**dadurch gekennzeichnet,**
**dass** die Kieselsäurefraktionen durch Fällung von Silikat mit einer Säure hergestellt, die Filterkuchen oder bereits getrocknete Kieselsäure verflüssigt und die so erhaltenen Suspensionen gemischt werden.

**19.** Verfahren nach einem der Ansprüche 11 bis 14,
**dadurch gekennzeichnet,**
**dass** eine oder mehrere Kieselsäurefraktionen aus einer pyrogenen Kieselsäure bestehen.

**20.** Verfahren nach einem der Ansprüche 11 bis 15 und 19,
**dadurch gekennzeichnet,**
**dass** die Kieselsäurefraktionen im trockenen Zustand gemischt werden.

**21.** Verwendung der Kieselsäure nach einem der Ansprüche 1 bis 10, in Zahnpasta, Zahnpflegemittel oder Schleifpasten.